# EUROPÄISCHE PATENTSCHRIFT

(11) **EP 3 488 758 B1**
(45) Veröffentlichungstag und Bekanntmachung des Hinweises auf die Patenterteilung: **14.07.2021**
(21) Anmeldenummer: 18000819.5
(22) Anmeldetag: 22.10.2018
(51) Int. Cl.: A61B 1/00, A61B 1/07, A61B 1/267

(54) **LARYNGOSKOPSPATEL UND VERFAHREN ZUM HERSTELLEN EINES LARYNGOSKOPSPATELS**
LARYNGOSCOPE SPATULA AND METHOD FOR MANUFACTURING A LARYNGOSCOPE SPATULA
SPATULE DE LARYNGOSCOPE ET PROCEDE POUR LA FABRICATION DE SPATULE DE LARYNGOSCOPE

(30) Priorität: 28.11.2017 DE 102017010987
(43) Veröffentlichungstag der Anmeldung: 29.05.2019
(73) Patentinhaber: Karl Storz SE & Co. KG, 78532 Tuttlingen (DE)
(72) Erfinder: Krause, Bernd, 78532 Tuttlingen (DE)
(74) Vertreter: Weidner Stern Jeschke

(56) Entgegenhaltungen:
- EP-A1- 3 127 645
- DE-B3-102012 204 178
- US-A- 3 986 854
- US-A- 4 958 624
- US-B1- 6 248 061

## Beschreibung

Die vorliegende Erfindung betrifft einen Laryngoskopspatel nach dem Oberbegriff von Anspruch 1, insbesondere einen Laryngoskopspatel nach Miller, sowie ein Verfahren zum Herstellen eines solchen Laryngoskopspatels.

Medizinische Instrumente, insbesondere invasive medizinische Instrumente, die zum Eindringen in einen menschlichen oder tierischen Körper bestimmt sind, unterliegen besonderen Anforderungen. Häufig werden derartige Instrumente aus Gründen der mechanischen und thermischen Belastbarkeit unter Verwendung metallischer Werkstoffe hergestellt. Insbesondere die Materialien, die die äußeren Oberflächen des Instruments bilden, müssen biokompatibel sein. Sofern das medizinische Instrument aus konstruktiven Gründen aus einer Mehrzahl einzelner Bauteile zusammengesetzt ist, ist eine dauerhafte Verbindung der Bauteile miteinander erforderlich. Die Verbindung muss dabei sowohl die notwendige Festigkeit als auch eine ausreichende Beständigkeit gegen chemische und thermische Einflüsse aufweisen. Auch die durch die Verbindung entstehenden äußeren Oberflächen des Instruments, wie Löt- oder Schweißnähte, müssen die Anforderungen der Biokompatibilität erfüllen. Diese Anforderungen gelten in erhöhtem Maße für wiederverwendbare medizinische Instrumente, die zur Reinigung und Sterilisation geeignet sein müssen, wobei sie chemisch aggressiven Substanzen sowie beim Autoklavieren einer erhöhten Temperatur und einem erhöhten Druck ausgesetzt sind.

Aus US 6,248,061 B1 ist ein Laryngoskopspatel nach Miller bekannt, der einen im Wesentlichen geraden Spatelabschnitt und einen nahe dem proximalen Ende des Spatelabschnitts angeordneten Basisabschnitt aufweist, an dem ein Handgriff angesetzt werden kann. Auf einer dem Basisabschnitt gegenüberliegenden Seite ist ein Saugrohr durch Schweißen fest auf einer äußeren Oberfläche des Spatelabschnitts befestigt. Schweißnähte sind jedoch in der Regel nicht vollständig lückenfrei, so dass eine ausreichende Reinigung und Sterilisation, die für eine Wiederverwendung notwendig ist, erschwert ist.

In US 3,986,854 ist ein Laryngoskop offenbart, das einen Handgriff und ein mit diesem verbundenes Spatelblatt aufweist. Als Beleuchtungsmittel ist ein Glasstab vorgesehen, der abschnittsweise in einem Schutzrohr aufgenommen ist. Ein Abschnitt des Schutzrohrs, der entlang des Spatelblatts verläuft, kann mit dem Spatelblatt und/oder den Wänden einer Öffnung des Spatelblatts durch Löten verbunden sein, um sicherzustellen, dass das Beleuchtungsmittel sicher gehalten wird.

Gemäß EP 3 127 645 A1, welches Dokument hiermit durch Bezugnahme in die vorliegende Anmeldung aufgenommen wird, weist ein Laryngoskopspatel ein Grundblatt und ein Deckblatt auf, die einen längs erstreckten Hohlraum ausbilden, der durch mindestens eine durch ein Lotmaterial gebildete Lötnaht fluiddicht begrenzt ist, wobei das Lotmaterial ein Eisenbasislot ist. Der Hohlraum dient zur Aufnahme optischer und/oder elektronischer Bauelemente. Zur Herstellung des Laryngoskopspatels werden das Grundblatt und das Deckblatt durch Laser-Punktschweißen miteinander verbunden, und Eisenbasislot wird in pastöser Form in den Hohlraum, der durch das Grundblatt und das Deckblatt gebildet wird, eingebracht. Der Lötverbund wird in einen Ofen eingebracht und auf eine Löttemperatur erwärmt. In weiteren Herstellungsschritten können optische und elektronische Bauelemente in den Hohlraum eingeführt werden. Die in EP 3 127 645 A1 beschriebene Bauart eines Laryngoskopspatels sowie das beschriebene Herstellungsverfahren sind für einen Laryngoskopspatel nach Miller nicht optimal.

In der nicht gattungsgemäßen Patentschrift DE 10 2012 204 178 B3 ist ein Mikrostrukturbauteil offenbart, das abwechselnd aufeinander gestapelte mikrostrukturierte Platten und unstrukturierte Platten aufweist, wobei die mikrostrukturierten Platten ein Wellenprofil aufweisen, das jeweils im Scheitelbereich rillenförmige Einbuchtungen aufweist, wobei Lotverbindungen zwischen den mikrostrukturierten und unstrukturierten Platten bestehen, die die rillenförmigen Einbuchtungen ausfüllen.

Es ist Aufgabe der vorliegenden Erfindung, einen verbesserten Laryngoskopspatel anzugeben, insbesondere einen Laryngoskopspatel nach Miller, der auf einfache und prozesssichere Weise herstellbar ist, sowie ein einfaches und prozesssicheres Verfahren zum Herstellen eines solchen Laryngoskopspatels.

Diese Aufgabe wird durch eine Vorrichtung gemäß Anspruch 1 sowie durch ein Verfahren gemäß Anspruch 8 gelöst.

Vorteilhafte Weiterbildungen der Erfindung ergeben sich aus den Unteransprüchen.

Ein erfindungsgemäßer Laryngoskopspatel umfasst ein Grundblatt und ein fest mit dem Grundblatt verbundenes Rohr, das zumindest abschnittsweise an einer Außenseite des Grundblatts angeordnet ist und sich im Wesentlichen in einer Längsrichtung des Grundblatts erstreckt. Der Laryngoskopspatel ist vorzugsweise ein Laryngoskopspatel nach Miller, bei dem das Grundblatt im Wesentlichen geradlinig ausgebildet ist und wobei das Grundblatt den eigentlichen Spatelteil des Laryngoskopspatels bildet. Das Grundblatt weist eine Außenseite und eine Innenseite auf, wobei die Außenseite zum Anlegen an bzw. zum Rückhalten von Gewebe und die Innenseite zur Gewährleistung der Sicht eines Benutzers in die Luftwege des Patienten dienen kann. Hierfür kann das Grundblatt in einem Querschnitt gekrümmt ausgebildet sein und einen in Längsrichtung durchgehenden Innenraum aufweisen. An einem proximalen (d.h. benutzernahen) Ende des Grundblatts kann ein Spatelkopf angeordnet sein, der zum Verbinden mit einem Handgriff dient. Das distale (d.h. benutzerferne) Ende des Grundblatts ist vorzugsweise atraumatisch abgerundet.

Das mit dem Grundblatt fest verbundene Rohr ist insbesondere ein Lichtkanalrohr zur Aufnahme von Lichtleitern, die das von einer Lichtquelle erzeugte Beleuchtungslicht vom proximalen Endbereich des Laryngoskopspatels zum distalen Endbereich leiten. Das Rohr kann aber auch beispielsweise zur Aufnahme elektrischer Leitungen oder als Saugrohr ausgebildet sein. Das Rohr kann eine im Wesentlichen zylindrische Form haben; insbesondere ist das Rohr umfangsseitig allseitig geschlossen und weist beispielsweise lediglich an seinem distalen und seinem proximalen Ende Öffnungen auf. Vorzugsweise reicht das Rohr proximalseitig an oder in den Spatelkopf hinein. Das Rohr weist insbesondere eine kleinere Querschnittsdimension als das Grundblatt auf, beispielsweise kann ein Durchmesser des Rohrs deutlich kleiner als eine Breite des Grundblatts sein.

Erfindungsgemäß weist das Rohr an seiner dem Grundblatt zugewandten Seite eine in Längsrichtung verlaufende Sicke auf, die mit der Außenseite des Grundblatts einen längs erstreckten Hohlraum bildet. Die Sicke kann sich als rinnenförmige Vertiefung in der Wand des Rohrs über die gesamte Länge des Rohrs oder nur über einen Abschnitt des Rohrs erstrecken. Insbesondere weist das Rohr im Bereich der Sicke in einem Querschnitt einen konkaven Umfangsabschnitt auf und hat im Übrigen eine konvexe Umfangslinie, wobei ein Krümmungsradius der Umfangslinie innerhalb der Sicke kleiner ist als ein Außenradius des Grundblatts in dem der Sicke zugewandten Bereich der Außenseite, sofern dieser im Querschnitt konvex ist.

Weiterhin erfindungsgemäß ist in den beiderseitigen seitlichen Randbereichen der Sicke das Rohr jeweils durch eine Lötnaht mit dem Grundblatt verbunden. Das Rohr bildet somit in den beiden Randbereichen der Sicke des Rohrs mit der Außenseite des Grundblatts jeweils einen längs erstreckten Lötspalt, der durch jeweils eine Lötnaht gefüllt ist, wodurch das Rohr fest mit dem Grundblatt verbunden ist. Insbesondere bilden die Längsränder der Sicke des Rohrs Fügebereiche, die mit entsprechenden Fügebereichen der Außenseite des Grundblatts zusammenwirken, um jeweils einen Lötspalt zu bilden, der durch ein Lotmaterial zumindest teilweise ausgefüllt ist. Der zwischen dem Rohr und dem Grundblatt ausgebildete Hohlraum ist dabei zwischen den Fügebereichen bzw. den Lötspalten angeordnet. Das Grundblatt und das Rohr können weitere Abschnitte aufweisen, in denen das Rohr nicht an der Außenseite des Grundblatts angeordnet und/oder das Rohr nicht über die in Längsrichtung verlaufenden Lötnähte mit dem Grundblatt verbunden ist. Vorzugsweise bestehen zumindest in den Fügebereichen sowohl das Grundblatt als auch das Rohr aus Edelstahl.

Dadurch, dass das Rohr mit einer längs erstreckten Sicke an der Außenseite des Grundblatts anliegt, wird ein längs erstreckter Hohlraum gebildet, der bei der Herstellung des Laryngoskopspatels zur Aufnahme von Lotmaterial zur Verfügung steht, das beim Lötvorgang die im Randbereich der Sicke gebildeten Lötspalte ausfüllt. Dies ermöglicht es, zumindest einen Teil des Lotmaterials innerhalb des Hohlraums angrenzend an die Lötspalte anzuordnen, wodurch auf einfache Weise die Prozesssicherheit des Lötvorgangs verbessert werden kann. Insbesondere kann dadurch, dass das Lotmaterial zumindest teilweise innerhalb des Hohlraums angeordnet werden kann, erreicht werden, dass das Lotmaterial beim Lötvorgang durch Kapillarkräfte in die Lötspalte gezogen wird und diese weitgehend gleichmäßig ausfüllt, so dass eine lückenfreie Lötnaht entsteht. Hierdurch kann auf einfache und prozesssichere Weise eine feste Verbindung des Rohrs mit dem Grundblatt gewährleistet werden, wobei zudem die Dichtigkeit des Rohrs selbst gewährleistet bleibt, dessen Innenraum beispielsweise zur Aufnahme von Lichtleitern zur Verfügung steht.

Vorzugsweise wird die Lötnaht durch ein Eisenbasislot gebildet, d.h. durch ein Lotmaterial auf Eisenbasis. Ein Eisenbasislot enthält beispielsweise etwa 28 bis 35 Gewichts-% Eisen sowie weitere Bestandteile, die in geringeren Gewichtsanteilen als Eisen enthalten sind. Ein solches Eisenbasislot, das gemäß EN ISO 3677 als B-FeCrNiSiP-1027/1097 bezeichnet wird, wird beispielsweise von der Fa. Innobraze (Esslingen, DE) unter der Bezeichnung ML 7813/S angeboten. Eisenbasislot ist biokompatibel, rostfrei und weist günstige Fließeigenschaften auf, wobei ein Verchromen nicht notwendig ist. Ferner kann es durch Verwendung von Eisenbasislot auf einfache Weise erreicht werden, dass die Lötnähte weitgehend lückenfrei und fluiddicht ausgebildet sind. Ebenso kann mit Eisenbasislot eine besonders glatte Oberfläche der Lötnähte erzeugt werden, wobei ggf. zusätzlich eine Glättung und/oder ein Überschweißen der Lötnähte, etwa durch Plasmaschweißen, vorgesehen sein kann. Dadurch, dass die Lötnaht aus Eisenbasislot besteht, wird auf einfache und sichere Weise eine dauerhafte Verbindung zwischen dem Grundblatt und dem Rohr ermöglicht, wobei zugleich die Anforderungen, die an medizinische Instrumente und insbesondere an Laryngoskopspatel gestellt sind, erfüllt werden können.

In vorteilhafter Weise sind das Rohr mit der längs erstreckten Sicke und die Außenseite des Grundblatts derart geformt, dass das Volumen des Hohlraums zumindest zur Aufnahme einer solchen Menge an Lotmaterial geeignet ist, dass dies zum Ausbilden der beiden Lötnähte ausreicht, insbesondere zum Ausbilden lückenloser Lötnähte. Dadurch wird es ermöglicht, dass die gesamte benötigte Menge an Lotmaterial innerhalb des Hohlraums angeordnet wird. Dies ist besonders vorteilhaft bei der Verwendung von Eisenbasislot als Lotmaterial. Hierdurch kann auf einfache Weise eine sichere Verbindung des Rohrs mit dem Grundblatt und die Ausbildung lückenfreier und glatter Lötnähte gewährleistet werden. Hierdurch kann die Prozesssicherheit beim Lötvorgang weiter verbessert werden.

Vorzugsweise ist das Grundblatt als langerstreckte, näherungsweise teilzylindrische Hohlform ausgebildet, beispielsweise näherungsweise halbzylindrisch oder auch etwa in Form eines Hohlzylinders, der einen durchgehenden Längsschlitz aufweist. Das Grundblatt bildet somit einen durchgehenden, beispielsweise teilzylindrisch geformten Innenraum, der bei der Verwendung des Laryngoskopspatels zum Ermöglichen einer ungehinderten Sicht und zum Aufnehmen etwa eines Intubationsschlauchs dienen kann. In einem distalen Endbereich kann das Grundblatt einen Durchbruch aufweisen, durch den ein distaler Abschnitt des Rohrs in den Innenraum des Grundblatts ragt, um beispielsweise eine Beleuchtung des durch den Innenraum betrachteten Körperbereichs des Patienten zu ermöglichen. Weiter ist gemäß dieser Ausführungsform, die insbesondere einen Laryngoskopspatel nach Miller darstellt, in einem proximalen Endbereich des Grundblatts ein Spatelkopf angeordnet, in den das proximale Ende des zumindest abschnittsweise an der Außenseite des Grundblatts angeordneten Rohrs mündet. Vorzugsweise ist der Spatelkopf an die Außenseite des Grundblatts angesetzt. Der Spatelkopf ist zum Ansetzen eines Handgriffs ausgebildet, der beispielsweise durch eine Rastverbindung mit dem Spatelkopf verbunden werden kann. Eine solche Ausgestaltung des Laryngoskopspatels ermöglicht es, innerhalb des Spatelkopfs zugleich eine Verbindung des Innenraums des Rohrs zu einer externen Versorgungseinrichtung, die an den Handgriff angeschlossen werden kann, zu schaffen. So kann beispielsweise der Spatelkopf zum Einkoppeln von Beleuchtungslicht, das von einer im Handgriff angeordneten oder an diesen angeschlossenen Lichtquelle erzeugt wird, in innerhalb des Rohrs geführte Lichtleiter ausgebildet sein.

Gemäß einer besonders bevorzugten Ausführungsform der Erfindung weist das Grundblatt außenseitig eine längs erstreckte Sicke auf, wobei das Rohr und das Grundblatt derart zueinander angeordnet sind, dass die jeweiligen Sicken einander zugewandt sind. Das Rohr ist insbesondere zumindest abschnittsweise an oder in die Sicke des Grundblatts angesetzt bzw. eingesetzt. Insbesondere bilden die Sicke des Rohrs und die Sicke des Grundblatts gemeinsam den Hohlraum aus. Hierdurch kann auf besonders einfache Weise ein Hohlraum mit einem ausreichenden Volumen und einer zum Einbringen des Lotmaterials geeigneten Form geschaffen werden, wobei zugleich die Lötspalte gebildet werden, in denen die Lötnähte zum Verbinden des Rohrs mit dem Grundblatt entstehen. Zudem kann hierdurch erreicht werden, dass weder der verbleibende Innenraum des Rohrs noch der Innenraum des Grundblatts durch die jeweiligen Sicken übermäßig eingeschränkt werden.

Gemäß einer bevorzugten Ausführungsform der Erfindung ist eine Breite der Sicke des Rohrs zumindest näherungsweise gleich einer Breite der Sicke des Grundblatts. Die jeweilige Breite ist dabei quer zur Längserstreckung des Rohrs bzw. des Grundblatts gemessen. Gemäß dieser Ausführungsform sind somit die Randbereiche der beiden Sicken aufeinandergesetzt, und die Lötspalte werden zwischen den einander entsprechenden Randbereichen der beiden Sicken gebildet. Das Grundblatt und das Rohr haben dabei insbesondere linienförmige Fügebereiche. Hierdurch wird eine einfache und prozesssichere Ausführung des Lötvorgangs ermöglicht, wobei besonders wenig Lotmaterial benötigt wird.

Gemäß einer weiteren bevorzugten Ausführungsform der Erfindung ist die Breite der Sicke des Rohrs kleiner als die Breite der Sicke des Grundblatts, wobei die Breite jeweils quer zur Längserstreckung gemessen ist. Hierdurch wird es ermöglicht, das Rohr in die Sicke des Grundblatts einzulegen, wodurch die Montage weiter vereinfacht wird. Auch hierbei können die Fügebereiche beispielsweise linienförmig ausgebildet sein.

In besonders bevorzugter Weise ist ein Innenradius der Sicke des Grundblatts näherungsweise gleich einem Außenradius des Rohrs. Dabei ist der Innenradius der Sicke des Grundblatts der in einem Querschnitt gemessene Krümmungsradius der äußeren Oberfläche des Grundblatts innerhalb der Sicke, und der Außenradius des Rohrs ist der in einem Querschnitt gemessene Krümmungsradius der Oberfläche des Rohrs in den der Sicke des Rohrs jeweils benachbarten Bereichen. In dem Fall, dass das Rohr einen kreisförmigen Umfang mit einem durch die Sicke gebildeten konkaven Abschnitt hat, ist der Innenradius der Sicke des Grundblatts näherungsweise gleich dem Radius des kreisförmigen Umfangs des Rohrs. Hierbei ist es besonders vorteilhaft, wenn die Breite der Sicke des Rohrs kleiner als die Breite der Sicke des Grundblatts ist. Gemäß dieser Ausführungsform liegt das Rohr somit derart in der Sicke des Grundblatts, dass der Sicke des Rohrs benachbarte Wandbereiche des Rohrs noch innerhalb der Sicke des Grundblatts liegen und, da die jeweiligen Radien näherungsweise gleich sind, einen etwa gleichmäßigen Lötspalt bilden. In diesem Fall sind die jeweiligen Fügebereiche, zwischen denen die Lötnähte ausgebildet sind, flächige Bereiche. Hierdurch kann eine besonders zuverlässige und fluiddichte Verbindung zwischen dem Rohr und dem Grundblatt geschaffen werden.

Gemäß einem erfindungsgemäßen Verfahren zum Herstellen eines Laryngoskopspatels werden in einem ersten Schritt ein Grundblatt, das außenseitig eine in Längsrichtung des Grundblatts verlaufende Sicke aufweisen kann, und ein Rohr, das zumindest abschnittsweise eine in Längsrichtung verlaufende Sicke aufweist, bereitgestellt. Das Grundblatt und das Rohr sind insbesondere wie oben beschrieben ausgebildet.

In einem zweiten Schritt wird das Rohr am Grundblatt zum Ausbilden eines Hohlraums zwischen der Sicke des Rohrs und der außenseitigen Oberfläche des Grundblatts gehalten, so dass beidseits des Hohlraums jeweils ein Lötspalt gebildet wird. Hierfür wird das Rohr insbesondere derart relativ zum Grundblatt gehalten, dass sich die jeweiligen Fügebereiche des Rohrs und des Grundblatts in einem geringen Abstand gegenüber stehen. Ferner wird Lotmaterial in dem Hohlraum angeordnet. Das Lotmaterial ist vorzugsweise ein Eisenbasislot in pastöser Form, beispielsweise das Eisenbasislot ML 7813/S der Fa. Innobraze (Esslingen, DE). Das Lotmaterial wird insbesondere derart angeordnet, dass es zumindest abschnittsweise auf beiden Seiten des Hohlraums sowohl an der Außenseite des Grundblatts als auch an der Sicke des Rohrs anliegt; das Lotmaterial kann den Hohlraum auch abschnittsweise ausfüllen. Hierdurch steht das Lotmaterial in Kapillarverbindung mit den beiden, zu beiden Seiten der Sicke des Rohrs gebildeten Lötspalten.

In einem dritten Schritt wird der auf diese Weise hergestellte Zusammenbau aus dem Grundblatt und dem Rohr sowie dem Lotmaterial, der auch als Lötverbund bezeichnet wird, in einen Ofen eingebracht und dort auf eine Löttemperatur erwärmt, d.h. insbesondere auf eine Temperatur, die mindestens der Schmelztemperatur des Lotmaterials entspricht, jedoch unter einer Schmelztemperatur des Materials des Grundblatts und des Rohrs liegt.

Der Lötverbund kann beispielsweise unter Vakuum oder Schutzgas auf die Löttemperatur erwärmt werden. Hierbei verflüssigt sich das Lotmaterial, etwaige Hilfsstoffe verdampfen und das flüssige Lot dringt aufgrund von Kapillarkräften in die zwischen dem Rohr und dem Grundblatt gebildeten Lötspalte ein und füllt diese aus.

Nachdem das Lot die beiden Lötspalte ausgefüllt hat, wird der Lötverbund abkühlen gelassen, bis das Lotmaterial erstarrt ist und das Rohr und das Grundblatt durch die Lötverbindung fest miteinander verbunden sind.

Das Verfahren kann weitere Schritte umfassen, insbesondere Schritte zum Herstellen des Grundblatts und des Rohrs und abschließende Fertigungsschritte nach dem Abkühlen des Laryngoskopspatels. So kann beispielsweise das Rohr aus einem zylindrischen Rohr hergestellt werden, in das abschnittsweise eine längs verlaufende Sicke eingedrückt wird. Das Grundblatt kann aus einem entsprechenden Rohling hergestellt werden, in dessen Außenseite ebenfalls abschnittsweise eine längs verlaufende Sicke eingedrückt werden kann. Ferner kann in an sich bekannter Weise das distale Ende des Grundblatts atraumatisch geformt werden. Weiterhin kann eine Vorbehandlung der miteinander zu verbindenden Bauteile bzw. der Fügebereiche vorgesehen sein. Nach dem Abkühlen des Lötverbunds können beispielsweise Lichtleiter in das Rohr eingeführt werden, vorzugsweise aus distaler Richtung, und das Rohr durch Verfüllen mit Klebstoff abgedichtet werden.

Durch das erfindungsgemäße Verfahren wird es auf einfache und prozesssichere Weise ermöglicht, einen Laryngoskopspatel herzustellen, beispielsweise einen Laryngoskopspatel nach Miller, der die an Laryngoskopspatel gestellten Anforderungen hinsichtlich der Anwendung, der Reinigung und der Sterilisation erfüllt.

Gemäß einer bevorzugten Ausführungsform des Verfahrens wird im zweiten Schritt das Rohr zunächst mit seinem distalen Abschnitt in einen in den distalen Endbereich des Grundblatts eingebrachten Durchbruch eingesteckt, danach mit einem mittleren Abschnitt am Grundblatt angelegt und sodann beispielsweise mit im proximalen Endbereich angeordneten zwei Laserschweißpunkten derart am Grundblatt gehalten, dass die Sicke des Rohrs mit der Oberfläche des Grundblatts den Hohlraum ausbildet und in den Randbereichen der Sicke die Lötspalte gebildet werden. Dabei ist es besonders vorteilhaft, wenn das Rohr in seinem distalen Endabschnitt gegenüber dem proximalen Abschnitt nach unten abgewinkelt ist; hierfür kann das Rohr in einem vorbereitenden Schritt entsprechend gebogen werden. In dem abgewinkelten distalen Endabschnitt muss das Rohr keine Sicke aufweisen. Wenn das Grundblatt selbst eine längs erstreckte Sicke aufweist, ist der Durchbruch in distaler Verlängerung dieser Sicke angeordnet, und das Rohr wird an oder in die Sicke des Grundblatts gelegt und in dieser Lage gehalten. Hierdurch kann das Herstellungsverfahren weiter vereinfacht werden.

Vorzugsweise wird in einem ersten Teilschritt des zweiten Schritts das Rohr zum Ausbilden des Hohlraums gehalten und sodann in einem zweiten Teilschritt das Lotmaterial in den Hohlraum eingebracht, insbesondere aus proximaler Richtung. Diese Ausführungsform des Verfahrens ist besonders einfach und prozesssicher. Alternativ kann das Lotmaterial zunächst in der Sicke des Rohrs und/oder auf der Oberfläche des Grundblatts angeordnet und sodann das Rohr an das Grundblatt angelegt werden.

In besonders bevorzugter Weise kann es vorgesehen sein, dass das Lotmaterial mit einer Dosiervorrichtung appliziert wird, beispielsweise mit einem Applikator, der eine Dosiereinrichtung und eine Spritzennadel umfasst. Insbesondere wird das Lotmaterial aus proximaler Richtung in den Hohlraum eingebracht und füllt einen proximalen Bereich des Hohlraums aus. Dass das gesamte benötigte Lotmaterial in den Hohlraum eingebracht wird, ist bei der Verwendung von Eisenbasislot besonders vorteilhaft. Die Dosiereinrichtung ist dabei zum Ausbringen der benötigten Menge an Lotmaterial eingerichtet, und die Spritzennadel ist zum Einbringen des Lotmaterials aus proximaler Richtung in den Hohlraum angepasst. Hierdurch wird auf einfache Weise das Anordnen der zum Erzeugen der Lötnähte benötigten Menge des Lotmaterials ermöglicht, was eine besonders sichere Durchführung des Verfahrens zum Erzeugen sauberer und glatter Lötnähte gestattet.

Gemäß einer bevorzugten Ausführungsform des Verfahrens wird im zweiten Schritt, nachdem das Rohr am Grundblatt gehalten und das Lotmaterial eingebracht worden ist, an einen proximalen Endbereich des Grundblatts insbesondere an die Außenseite des Grundblatts ein Spatelkopf angesetzt, wobei das Rohr in eine entsprechende Bohrung des Spatelkopfs eingesetzt werden kann. Der Spatelkopf kann beispielsweise ebenfalls mit Laserschweißpunkten am Grundblatt fixiert werden, und Lotmaterial an den dadurch entstandenen Lötspalten des Spatelkopfs angeordnet werden. Im dritten Schritt kann auf diese Weise auch eine Lötverbindung zwischen dem Spatelkopf und dem Grundblatt sowie dem Rohr erzeugt werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1a und 1b ein Ausführungsbeispiel eines erfindungsgemäßen Laryngoskopspatels in zwei unterschiedlichen Ansichten;
Fig. 2a bis 2c drei unterschiedliche Ansichten einer Variante des Ausführungsbeispiels gemäß Fig. 1a und 1b;
Fig. 3 einen Querschnitt durch das Lichtkanalrohr des Laryngoskopspatels gemäß Fig. 1a und 1b bzw. der Variante gemäß Fig. 2a bis 2c;
Fig. 4 eine Querschnittsansicht des Laryngoskopspatels gemäß Fig. 1a und 1b bzw. Fig. 2a bis 2c;
Fig. 5 einen Längsschnitt des Laryngoskopspatels gemäß Fig. 2a bis 2c.

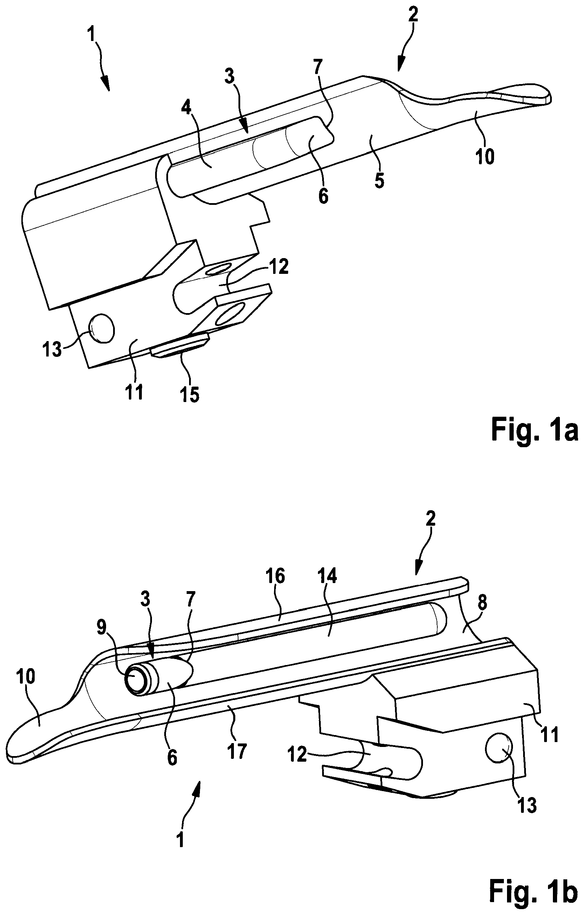

In Fig. 1a und 1b ist ein Ausführungsbeispiel eines erfindungsgemäßen Laryngoskopspatels in zwei Schrägansichten gezeigt. Der Laryngoskopspatel 1 ist als Laryngoskopspatel nach Miller ausgebildet und weist ein Grundblatt 2 auf, das näherungsweise halbzylindrisch geformt ist. Mit dem Grundblatt 2 ist ein Lichtkanalrohr 3 fest verbunden, das in einem mittleren Abschnitt 4 an einer Außenseite 5 des Grundblatts 2 angeordnet ist und parallel zur Längsrichtung des Grundblatts 2 verläuft. Ein distaler Abschnitt 6 des Lichtkanalrohrs 3 ist gegenüber dem proximalen Abschnitt 4 abgewinkelt und tritt durch einen Durchbruch 7 zur Innenseite 8 des Grundblatts 2 hindurch. Innerhalb des Lichtkanalrohrs 3 verlaufen Lichtleitfasern zur Weiterleitung von Beleuchtungslicht, die am distalen Ende des Lichtkanalrohrs 3 in einer Lichtaustrittsfläche 9 münden. Das distale Ende des Grundblatts 2 ist als löffelförmiger Fortsatz 10 ausgebildet. Die Kanten des Fortsatzes 10 und auch die übrigen Kanten des Grundblatts 2 sind atraumatisch abgerundet.

Am proximalen Ende des Grundblatts 2 ist ein Spatelkopf 11 angeordnet, der fest mit dem Grundblatt 2 verbunden ist. An den Spatelkopf 11 ist mit einer Quernut 12 und gefederten Rasten 13 ein nicht dargestellter Handgriff anschließbar, der dann etwa senkrecht vom Grundblatt 2 nach unten absteht. Entsprechend den in Fig. 1a und 1b gezeigten Ansichten wird hier und im Folgenden mit "unten" die Richtung bezeichnet, in der der Spatelkopf 11 und der Handgriff, wenn dieser angeschlossen ist, vom Grundblatt 2 abstehen, und mit "oben" die entgegengesetzte Richtung; "unten" und "oben" beziehen sich dabei nur auf die relative Lage der Bauteile zueinander, nicht auf die Ausrichtung des Laryngoskopspatels 1 bei dessen Anwendung. Bei dem gezeigten Ausführungsbeispiel ist dementsprechend, aus proximaler Richtung gesehen, das Grundblatt 2 nach links offen, während rechts das Lichtkanalrohr 3 angeordnet ist.

Das Grundblatt 2 weist eine in Längsrichtung verlaufende, in die Außenseite 5 eingedrückte Sicke auf, in die das Lichtkanalrohr 3 eingelegt ist und die auf der Innenseite 8 als längserstreckter Wulst 14 erkennbar ist (s. Fig. 1b). Die Sicke bzw. der Wulst 14 teilt das Grundblatt 2 in einen oberen Flügel 16 und einen unteren Flügel 17. Ein proximaler Abschnitt des Lichtkanalrohrs 3 ist in den Spatelkopf 11 eingesetzt. Innerhalb des Spatelkopfs 11 verlaufen die im Lichtkanalrohr 3 geführten Lichtleitfasern gekrümmt und münden in eine Lichtanschlussfläche 15. An die Lichtanschlussfläche 15 kann eine entsprechende Lichtkupplungsfläche des Handgriffs angesetzt werden, um Beleuchtungslicht in die Lichtleitfasern des Lichtkanalrohrs 3 einzukoppeln, die das Beleuchtungslicht zur Lichtaustrittsfläche 9 weiterleiten.

Das Grundblatt 2 und das Lichtkanalrohr 3 bestehen jeweils aus Edelstahl und sind durch Löten mit Eisenbasislot miteinander verbunden. Auch der Spatelkopf 11 ist aus Edelstahl gefertigt und durch Löten mit Eisenbasislot mit dem Grundblatt 2 und dem Lichtkanalrohr 3 verbunden.

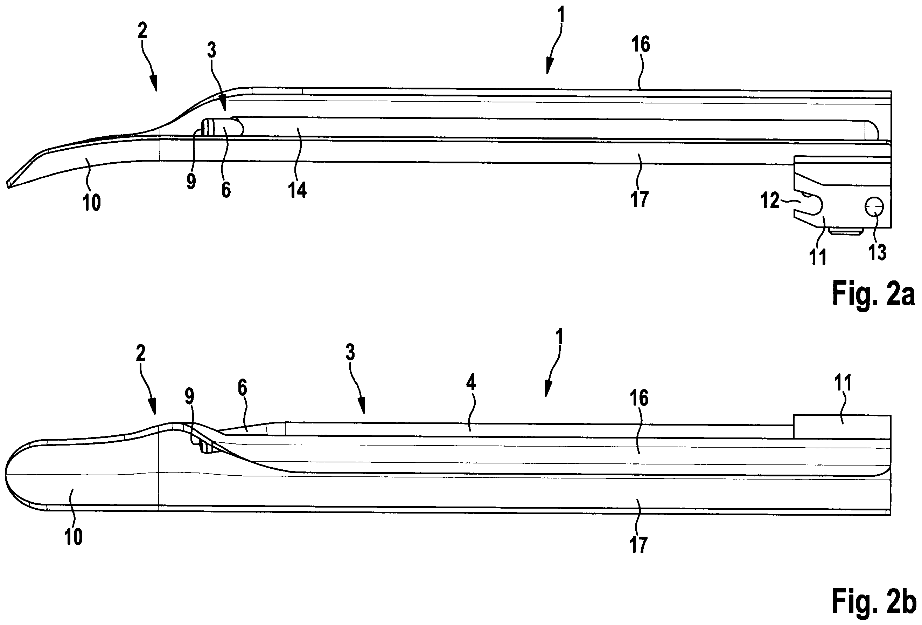

In Fig. 2a bis 2c sind drei unterschiedliche Ansichten einer Variante des zuvor beschriebenen Ausführungsbeispiels dargestellt. Diese Variante unterscheidet sich von der in Fig. 1a und 1b gezeigten Variante lediglich durch die Länge des Grundblatts 2 sowie die entsprechende Länge des mittleren Abschnitts 4 des Lichtkanalrohrs 3. Im Übrigen sind die beiden Varianten gleich ausgebildet. Die Variante der Fig. 1a und 1b kann beispielsweise ein Laryngoskopspatel für Kinder sein (Größe 0 oder 1), während die in Fig. 2a bis 2c dargestellte Variante ein Laryngoskopspatel für Erwachsene ist (Größe 3 oder 4).

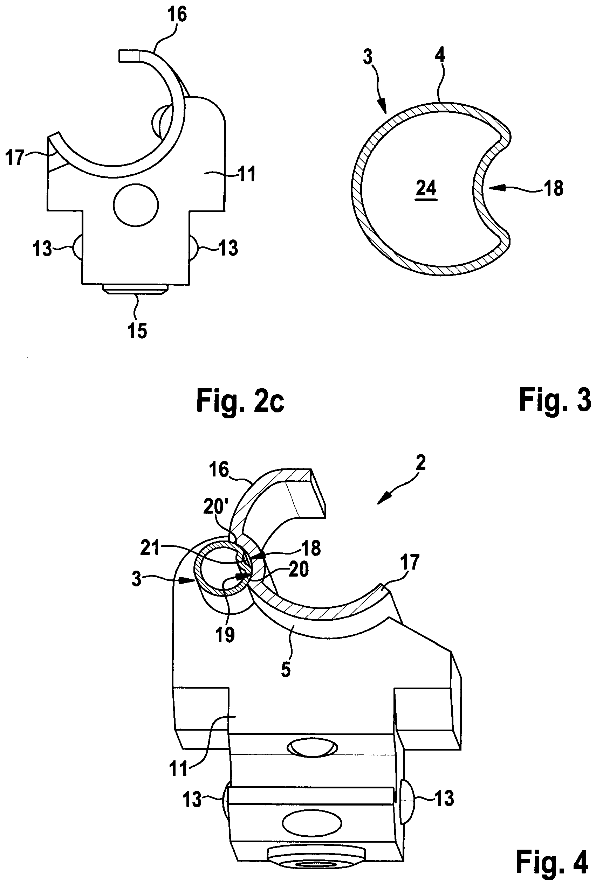

Fig. 2a zeigt eine Seitenansicht des Laryngoskopspatels 1 von links gesehen, Fig. 2b eine Seitenansicht von oben gesehen und Fig. 2c eine Ansicht aus proximaler Richtung. In Fig. 2a ist gezeigt, dass der löffelförmige Fortsatz 10 leicht nach unten abgewinkelt ist. Wie in Fig. 2b zu erkennen ist, ist der distale Abschnitt 6 des Lichtkanalrohrs 3 mit der Lichtaustrittsfläche 9 leicht nach links abgewinkelt. Hierdurch kann ein Bereich ausgeleuchtet werden, der distalseitig des Laryngoskopspatels 1 und leicht nach links versetzt angeordnet ist. In Fig. 2c ist dargestellt, dass der obere Flügel 16 etwas schmaler ist als der untere Flügel 17; beide zusammen bilden näherungsweise einen Halbzylinder und nehmen, von einer Längsachse des Halbzylinders aus gemessen, einen Bogen von etwas mehr als 180° ein. Der obere Flügel 16 ist im oberen Bereich etwas stärker nach innen gekrümmt.

In Fig. 3 ist ein Querschnitt durch das Lichtkanalrohr 3 in dessen mittlerem Abschnitt 4 dargestellt. Das Lichtkanalrohr 3 ist im Wesentlichen zylindrisch ausgebildet mit einer über den größten Teil des Umfangs kreisförmigen Außenkontur, wobei an einer Seite eine Sicke 18 eingedrückt ist. Der Innenraum 24, der durch die Sicke 18 nur geringfügig eingeschränkt wird, dient zur Aufnahme der Lichtleitfasern. Der abgewinkelte distale Abschnitt 6 des Lichtkanalrohrs 3 weist keine Sicke auf (s. Fig. 1a bis 2b).

Im montierten Zustand ist das Lichtkanalrohr 3 in die zuvor erwähnte Sicke des Grundblatts 2 eingesetzt, und zwar derart, dass die Sicke 18 des Lichtkanalrohrs 3 dem Grundblatt 2 bzw. der Sicke des Grundblatts 2 zugewandt ist. Dies ist in der Querschnittsansicht der Fig. 4 dargestellt, die schräg aus distaler Richtung gesehen einen Schnitt durch das Grundblatt 2 und das Lichtkanalrohr 3 zeigt. Wie in Fig. 4 gezeigt ist, hat die Sicke 18 des Lichtkanalrohrs 3 eine geringere Breite als die Sicke 19 des Grundblatts. Der Krümmungsradius der Oberfläche des Grundblatts 2 im Inneren der Sicke 19 entspricht etwa dem Radius der kreisförmigen Außenkontur des Lichtkanalrohrs 3, so dass das in die Sicke 19 des Grundblatts 2 eingelegte Lichtkanalrohr 3 zu beiden Seiten der Sicke 18 flächig an dem Grundblatt 2 anliegt bzw. zu beiden Seiten der Sicke 18 jeweils einen etwa gleichmäßig breiten Lötspalt 20, 20' bildet. Die beidseits der Sicke 18 liegenden Bereiche der Oberfläche des Lichtkanalrohrs 3 und die diesen gegenüber liegenden Bereiche innerhalb der Sicke 19 des Grundblatts 2 dienen als Fügebereiche. Die Sicke 18 des Lichtkanalrohrs 3 bildet mit dem übrigen Teil der Sicke 19 des Grundblatts 2 einen Hohlraum 21.

Der Hohlraum 21 dient zur Aufnahme des Lotmaterials vor dem Lötvorgang. Die beiden Lötspalte 20, 20' haben eine solche Weite, dass beim Lötvorgang das Eisenbasislot, das als Lotmaterial verwendet wird, in die Lötspalte 20, 20' eindringt, sich über die gesamte Länge der Lötspalte 20, 20' verteilt und diese ausfüllt, beispielsweise eine Weite von ca. 0,03 bis 0,05 mm. Hierdurch wird nicht nur eine feste Verbindung des Lichtkanalrohrs 3 mit dem Grundblatt 2 geschaffen, sondern es entsteht an der Außenseite der Lötspalte 20, 20' eine Lötnaht mit einer lückenfreien, glatten Oberfläche. Insbesondere wird ausreichend Lötmaterial in den Hohlraum 21 eingebracht, so dass auch der distale Abschnitt 6 des Lichtkanalrohrs 3 in dem Durchbruch 7 von einer Lötnaht umgeben ist (s. Fig. 1a und 1b). Hierdurch wird der Hohlraum 21 zugleich distalseitig abgeschlossen. Ferner wird der Spatelkopf 11 durch Löten mit dem Grundblatt 2 und dem Lichtkanalrohr 3 verbunden, wodurch der Hohlraum 21 auch proximalseitig verschlossen werden kann.

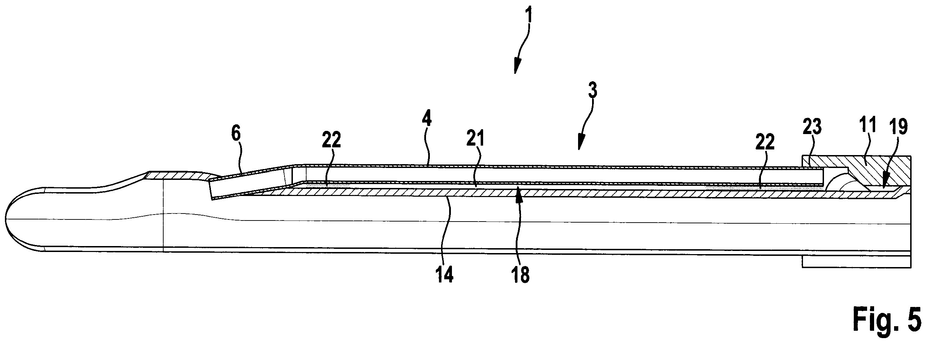

In dem in Fig. 5 gezeigten Längsschnitt des Laryngoskopspatels 1 ist dargestellt, dass der Hohlraum sich vom proximalen Ende des Lichtkanalrohrs 3 bis zum Übergang zwischen dem mittleren Abschnitt 4 und dem abgewinkelten distalen Abschnitt 6 des Lichtkanalrohrs 3 erstreckt. Dabei ist symbolisch das vor dem Lötvorgang eingebrachte Lotmaterial 22 angedeutet. Gemäß Fig. 5 kann das Lotmaterial in einem distalen und in einem proximalen Bereich des Hohlraums 21 angeordnet sein, in der Regel reicht es aber aus, das Lotmaterial in den proximalen Bereich des Hohlraums 21 einzubringen. In Fig. 5 ist auch zu erkennen, dass das Lichtkanalrohr 3 mit seinem proximalen Abschnitt 23 in den Spatelkopf 11 hineinragt und dass die Sicke 19 des Grundblatts 2 bzw. der Wulst 14 in proximaler Richtung etwas über das proximale Ende des Lichtkanalrohrs 3 hinausreicht.

Zur Herstellung des Laryngoskopspatels 1 werden gemäß einem Ausführungsbeispiel des erfindungsgemäßen Verfahrens zunächst das Grundblatt 2, das Lichtkanalrohr 3 und der Spatelkopf 11 bereitgestellt. Das Lichtkanalrohr 3 wird mit seinem abgewinkelten distalen Abschnitt in den Durchbruch 7 des Grundblatts 2 eingeschoben und sodann in die Sicke 19 des Grundblatts 2 eingelegt und in dieser Position, in der beidseits der Sicke 18 des Lichtkanalrohrs zur Sicke 19 des Grundblatts 2 jeweils ein Lötspalt 20, 20' besteht, in oder nahe seinem proximalen Abschnitt 23 mit zwei Laserschweißpunkten am Grundblatt 2 angeheftet. Sodann wird Eisenbasislot als Lotpaste mit einem Applikator, der eine Dosiereinrichtung und eine Spritzennadel umfasst, aus proximaler Richtung in den zwischen der Sicke 18 des Lichtkanalrohrs 3 und dem Grundblatt 2 gebildeten Hohlraum eingebracht, bis dieser in einem proximalen Bereich mit dem Lotmaterial ausgefüllt ist; optional kann auch ein distaler Bereich mit Lotmaterial ausgefüllt werden (s. Fig. 5). Weiter wird der Spatelkopf 11 an den proximalen Endbereich des Grundblatts 2 angesetzt, so dass der proximale Abschnitt 23 in den Spatelkopf 11 hineinreicht, und ebenfalls mit Laserschweißpunkten am Grundblatt 2 befestigt; ebenso kann Lotmaterial an den entsprechenden Lötspalten des Spatelkopfs 11 angeordnet werden.

Dieser Lötverbund wird in einen Ofen eingebracht und auf eine Löttemperatur von etwa 1120 °C erwärmt. Das Eisenbasislot verflüssigt sich dabei und fließt aufgrund der Kapillarwirkung in die Lötspalte 20, 20' sowie in entsprechende Lötspalte des Spatelkopfs 11. Ebenso wird der Spalt zwischen dem Durchbruch 7 und dem distalen Abschnitt 6 des Lichtkanalrohrs mit Lot ausgefüllt. Beim nachfolgenden Abkühlen, das beispielsweise innerhalb einer oder weniger Stunden erfolgen kann, erstarrt das Eisenbasislot in den Lötspalten und bildet Lötnähte, wodurch eine feste, haltbare und lückenlose Verbindung zwischen den miteinander zu verbindenden Bauteilen geschaffen wird. Die entstandenen Lötnähte weisen eine glatte Oberfläche auf, so dass in der Regel keine oder nur eine geringfügige Nachbehandlung notwendig ist.

Nach dem Abkühlen werden Lichtleiter aus distaler Richtung in das Lichtkanalrohr 3 und weiter in den Spatelkopf 11 eingeschoben. Schließlich werden durch Verfüllen mit Klebstoff und nachfolgende Bearbeitung die Lichtaustrittsfläche 9 und die Lichtanschlussfläche 15 geschaffen.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Laryngoskopspatel
- 2: Grundblatt
- 3: Lichtkanalrohr
- 4: Mittlerer Abschnitt
- 5: Außenseite
- 6: Distaler Abschnitt
- 7: Durchbruch
- 8: Innenseite
- 9: Lichtaustrittsfläche
- 10: Fortsatz
- 11: Spatelkopf
- 12: Quernut
- 13: Raste
- 14: Wulst
- 15: Lichtanschlussfläche
- 16: Oberer Flügel
- 17: Unterer Flügel
- 18: Sicke
- 19: Sicke
- 20, 20': Lötspalt
- 21: Hohlraum
- 22: Lotmaterial
- 23: Proximaler Abschnitt
- 24: Innenraum

## Patentansprüche

1. Laryngoskopspatel mit einem Grundblatt (2) und einem zumindest abschnittsweise an einer Außenseite (5) des Grundblatts (2) angeordneten und sich näherungsweise in einer Längsrichtung des Grundblatts (2) erstreckenden, fest mit dem Grundblatt (2) verbundenen Rohr, **dadurch gekennzeichnet, dass** das Rohr an seiner dem Grundblatt (2) zugewandten Seite zumindest abschnittsweise eine längs erstreckte Sicke (18) aufweist, die mit der Außenseite (5) des Grundblatts (2) einen längs erstreckten Hohlraum (21) bildet, wobei in den seitlichen Randbereichen der Sicke (18) das Rohr durch je eine Lötnaht mit dem Grundblatt (2) verbunden ist.

2. Laryngoskopspatel nach Anspruch 1, **dadurch gekennzeichnet, dass** die Lötnaht durch Eisenbasislot gebildet ist.

3. Laryngoskopspatel nach Anspruch 1 oder 2, **dadurch gekennzeichnet, dass** das Volumen des Hohlraums (21) mindestens zur Aufnahme einer zum Ausbilden der Lötnähte ausreichenden Menge an Lotmaterial (22) bemessen ist.

4. Laryngoskopspatel nach einem der vorhergehenden Ansprüche, **dadurch gekennzeichnet, dass** das Grundblatt (2) als langerstreckte, näherungsweise teilzylindrische Hohlform ausgebildet ist, wobei an einem proximalen Endbereich des Grundblatts (2) ein Spatelkopf (11) angeordnet ist, in den das Rohr mündet.

5. Laryngoskopspatel nach einem der vorhergehenden Ansprüche, **dadurch gekennzeichnet, dass** das Grundblatt (2) außenseitig eine längs erstreckte Sicke (19) aufweist und dass die Sicke (18) des Rohrs und die Sicke (19) des Grundblatts (2) einander zugewandt sind.

6. Laryngoskopspatel nach Anspruch 5, **dadurch gekennzeichnet, dass** eine Breite der Sicke (18) des Rohrs kleiner als oder näherungsweise gleich groß wie eine Breite der Sicke (19) des Grundblatts (2) ist.

7. Laryngoskopspatel nach Anspruch 5 oder 6, **dadurch gekennzeichnet, dass** ein Innenradius der Sicke (19) des Grundblatts (2) zumindest näherungsweise gleich einem Außenradius des Rohrs ist.

8. Verfahren zum Herstellen eines Laryngoskopspatels, wobei ein Grundblatt (2) und ein Rohr, das zumindest abschnittsweise eine längs erstreckte Sicke (18) aufweist, bereitgestellt werden, das Rohr derart am Grundblatt (2) gehalten wird, dass die Sicke (18) einer Außenseite (5) des Grundblatts (2) zugewandt ist und mit dieser einen längs erstreckten Hohlraum (21) ausbildet, ein Lotmaterial (22) in den Hohlraum (21) eingebracht wird und der Zusammenbau aus dem Grundblatt (2) und dem Rohr mit dem Lotmaterial (22) auf eine Löttemperatur erwärmt wird.

9. Verfahren nach Anspruch 8, **dadurch gekennzeichnet, dass** das Rohr dadurch am Grundblatt (2) gehalten wird, dass es mit einem distalen Abschnitt (6) in einen in einem distalen Endbereich des Grundblatts (2) vorhandenen Durchbruch (7) eingesteckt wird, mit einem mittleren Abschnitt (4) zum Ausbilden des Hohlraums (21) am Grundblatt (2) angelegt wird und mit mindestens einem Laserschweißpunkt fixiert wird.

10. Verfahren nach Anspruch 8 oder 9, **dadurch gekennzeichnet, dass** das Lotmaterial (22) nach dem Ausbilden des Hohlraums (21) in den Hohlraum (21) eingebracht wird.

11. Verfahren nach einem der Ansprüche 8 bis 10, **dadurch gekennzeichnet, dass** das Lotmaterial (22) mit einer Dosiervorrichtung appliziert wird.

12. Verfahren nach einem der Ansprüche 8 bis 11, **dadurch gekennzeichnet, dass** in einem proximalen Endbereich des Grundblatts (2) ein Spatelkopf (11) angesetzt und ein proximaler Abschnitt (23) des Rohrs in den Spatelkopf (11) eingesetzt wird.

## Claims

1. Laryngoscope spatula with a base sheet (2) and a tube, which is arranged at least in sections on an outer side (5) of the base sheet (2), extends approximately in a longitudinal direction of the base sheet (2) and is fixedly connected to the base sheet (2), **characterised in that** the tube comprises on its side facing the base sheet (2) at least in sections a longitudinally extended bead (18), which forms with the outer side (5) of the base sheet (2) a longitudinally extended cavity (21), the tube being connected in the lateral edge areas of the bead (18) to the base sheet (2) by a soldered seam in each case.

2. Laryngoscope spatula according to claim 1, **characterised in that** the soldered seam is formed by iron-based solder.

3. Laryngoscope spatula according to claim 1 or 2, **characterised in that** the volume of the cavity (21) is dimensioned at least to receive a sufficient quantity of solder material (22) to form the soldered seams.

4. Laryngoscope spatula according to any one of the preceding claims, **characterised in that** the base sheet (2) is formed as an elongated, approximately partly cylindrical hollow form, wherein a spatula head (11) is arranged on a proximal end area of the base sheet (2), into which head the tube opens.

5. Laryngoscope spatula according to any one of the preceding claims, **characterised in that** the base sheet (2) comprises a longitudinally extended bead (19) on the outer side and that the bead (18) of the tube and the bead (19) of the base sheet (2) face one another.

6. Laryngoscope spatula according to claim 5, **characterised in that** a width of the bead (18) of the tube is smaller than or approximately of the same size as a width of the bead (19) of the base sheet (2).

7. Laryngoscope spatula according to claim 5 or 6, **characterised in that** an inner radius of the bead (19) of the base sheet (2) is at least approximately equal to an outer radius of the tube.

8. Method for manufacturing a laryngoscope spatula, wherein a base sheet (2) and a tube, which comprises at least in sections a longitudinally extended bead (18), are provided, wherein the tube is held on the base sheet (2) in such a way that the bead (18) faces an outer side (5) of the base sheet (2) and forms with this a longitudinally extended cavity (21), wherein a solder material (22) is introduced into the cavity (21) and the assembly of the base sheet (2) and the tube is heated with the solder material (22) to a soldering temperature.

9. Method according to claim 8, **characterised in that** the tube is held on the base sheet (2), **in that** it is inserted with a distal section (6) into an opening (7) present in a distal end area of the base sheet (2), is placed with a central section (4) for forming the cavity (21) on the base sheet (2) and is fixed by at least one laser weld point.

10. Method according to claim 8 or 9, **characterised in that** the solder material (22) is introduced, after forming the cavity (21), into the cavity (21).

11. Method according to any one of claims 8 to 10, **characterised in that** the solder material (22) is applied with a metering device.

12. Method according to any one of claims 8 to 11, **characterised in that** a spatula head (11) is attached in a proximal end area of the base sheet (2) and a proximal section (23) of the tube is inserted into the spatula head (11).

## Revendications

1. Spatule de laryngoscope avec une lame de base (2) et un tube disposé au moins par endroits sur un côté extérieur (5) de la lame de base (2) et s'étendant approximativement dans un sens longitudinal de la lame de base (2), relié de manière solidaire à la lame de base (2), **caractérisée en ce que** le tube présente sur son côté tourné vers la lame de base (2) au moins par endroits une moulure (18) s'étendant longitudinalement, qui forme avec le côté extérieur (5) de la lame de base (2) un espace creux (21) s'étendant longitudinalement, dans laquelle le tube est relié à la lame de base (2) par respectivement un cordon de brasage dans des zones de bordure latérales de la moulure (18).

2. Spatule de laryngoscope selon la revendication 1, **caractérisée en ce que** le cordon de brasage est formé par un métal d'apport de brasage à base de fer.

3. Spatule de laryngoscope selon la revendication 1 ou 2, **caractérisée en ce que** le volume de l'espace creux (21) est dimensionné au moins pour recevoir une quantité de matériau de métal d'apport de brasage (22) suffisante pour réaliser les cordons de brasage.

4. Spatule de laryngoscope selon l'une quelconque des revendications précédentes, **caractérisée en ce que** la lame de base (2) est réalisée en tant que forme creuse allongée, approximativement en partie cylindrique, dans laquelle est disposée sur une zone d'extrémité proximale de la lame de base (2) une tête de spatule (11), dans laquelle débouche le tube.

5. Spatule de laryngoscope selon l'une quelconque des revendications précédentes, **caractérisée en ce que** la lame de base (2) présente côté extérieur une moulure (19) s'étendant longitudinalement, et que la moulure (18) du tube et la moulure (19) de la lame de base (2) sont tournées l'une vers l'autre.

6. Spatule de laryngoscope selon la revendication 5, **caractérisée en ce qu'**une largeur de la moulure (18) du tube est inférieure à ou approximativement aussi grande qu'une largeur de la moulure (19) de la lame de base (2).

7. Spatule de laryngoscope selon la revendication 5 ou 6, **caractérisée en ce qu'**un rayon intérieur de la moulure (19) de la lame de base (2) est au moins approximativement égal à un rayon extérieur du tube.

8. Procédé pour fabriquer une spatule de laryngoscope, dans lequel une lame de base (2) et un tube, qui présente au moins par endroits une moulure (18) s'étendant longitudinalement, sont fournis, le tube est maintenu de telle manière sur la lame de base (2) que la moulure (18) est tournée vers un côté extérieur (5) de la lame de base (2) et réalise avec celui-ci un espace creux (21) s'étendant longitudinalement, un matériau de métal d'apport de brasage (22) est introduit dans l'espace creux (21), et l'assemblage composé de la lame de base (2) et du tube avec le matériau de métal d'apport de brasage (22) est chauffé à une température de brasage.

9. Procédé selon la revendication 8, **caractérisé en ce que** le tube est maintenu sur la lame de base (2) **en ce qu'**il est emboîté par une section distale (6) dans un ajour (7) présent dans une zone d'extrémité distale de la lame de base (2), est placé sur la lame de base (2) par une section centrale (4) pour réaliser l'espace creux (21) et est fixé avec au moins un point de soudage au laser.

10. Procédé selon la revendication 8 ou 9, **caractérisé en ce que** le matériau de métal d'apport de brasage (22) est introduit dans l'espace creux (21) après la réalisation de l'espace creux (21).

11. Procédé selon l'une quelconque des revendications 8 à 10, **caractérisé en ce que** le matériau de métal d'apport de brasage (22) est appliqué avec un dispositif de dosage.

12. Procédé selon l'une quelconque des revendications 8 à 11, **caractérisé en ce qu'**une tête de spatule (11) est placée dans une zone d'extrémité proximale de la lame de base (2) et une section proximale (23) du tube est insérée dans la tête de spatule (11).
